# EUROPEAN PATENT SPECIFICATION

(11) **EP 1 666 014 B1**
(45) Date of publication and mention of the grant of the patent: **16.12.2009**
(21) Application number: 05020277.9
(22) Date of filing: 16.09.2005
(51) Int. Cl.: A61G 3/00, A61F 5/37

(54) **Wheelchair**
Rollstuhl
Fauteuil roulant

(30) Priority: 27.10.2004 JP 2004311720
(43) Date of publication of application: 07.06.2006
(73) Proprietor: TAKATA CORPORATION, Minato-ku, Tokyo 106-8510 (JP)
(72) Inventor: Hiruta, Teruhiko Takata Corp., Tokyo 106-8510 (JP); Sakano, Takashi Takata Corp., Tokyo 106-8510 (JP)
(74) Representative: Banzer, Hans-Jörg

(56) References cited:
- WO-A-02/091979
- FR-A- 504 711
- GB-A- 2 389 821
- US-A- 4 455 046
- US-A- 5 028 065
- US-A1- 2003 137 180
- US-A1- 2005 073 187
- PATENT ABSTRACTS OF JAPAN vol. 1998, no. 03, 27 February 1998 (1998-02-27) & JP 09 285500 A (SATOU NAGAYOSHI), 4 November 1997 (1997-11-04)

## Description

The present invention relates to a wheelchair which permits an occupant to move in his or her seated state and, more particularly, to a wheelchair suitably used to be put in a vehicle such as an automobile.

### [Background of the Invention]

Nowadays, wheelchairs are widely used as simple vehicles which are necessary for permitting an occupant such as an injured person with wounded legs or a handicapped person with impaired legs to move in the seated state. The structure of a commonly used wheelchair is a foldable structure which comprises a pipe frame, which rotatably supports main wheels and front auxiliary wheels and is openable and closable in the lateral direction, and a bottom sheet and a back sheet which are made of cloth and are stretched between pipes of the pipe frame.

Since such occupant on a wheelchair may be not able to brace his or her legs, the occupant hardly keeps balance on the bottom sheet and the back sheet while being seated in the wheelchair. Therefore, wheelchair is desired to have a function of holding the occupant's body securely when used such as outing or the like. For this, wheelchairs provided with a seat belt for holding the occupant's body have been conventionally proposed. (For example, see Japanese Patent Unexamined Publication 2000-316911)

Recently, automobiles provided with equipment for permitting a wheelchair with the occupant seated therein to be brought onto a cargo compartment of the automobile in order to save the occupant's effort of riding on a seat of the automobile from the wheelchair have become popular. However, the structure of the wheelchair as described in the aforementioned Japanese Patent Unexamined Publication 2000-316911 even with the seat belt can not restrain the occupant's body sufficiently against strong impact produced by sudden stop or collision of the automobile when the wheelchair is put in the automobile and thus can not ensure the safety of the occupant.

US 4,455,046, which represents the most relevant prior art, discloses a safety device for the vehicular transport of a person travelling in a wheelchair. The safety device includes one safety belt anchored to the wheelchair to hold the occupant in the wheelchair and an inertia reel for the safety belt.

WO 02/091979 A1 discloses a restraint for restraining a wheelchair occupant in a vehicle. The restraint includes a support structure carrying a seat squab and a movable seatback provided with a safety belt arrangement. When a wheelchair is secured, the seatback of the support structure is moved so as to be arranged adjacent a backrest of the wheelchair, so that the occupant of the wheelchair can use the safety belt arrangement provided on the seatback of the support structure.

US 5,028,065 discloses a wheelchair in which the surface of the seat, the back support and sidewalls are designed as a self supporting monocoque construction.

GB 2 389 821 A, US 2005/0073187 A1 and US 2003/0137180 A1 respectively disclose a vehicle seat which is provided with plural seat belts for restraining an occupant, which has plural seat belt retractors associated with the seat belts.

The object of the present invention is to provide a wheelchair capable of ensuring the safety of an occupant by securely restraining the occupant's body even against a collision of an automobile when the wheelchair is put in the automobile.

According to the present invention, this object is achieved by a wheelchair as defined in claim 1. The dependent claims define preferred and advantageous embodiments of the invention.

A first aspect for achieving the aforementioned object is a wheelchair comprising: seat belts capable of restraining the body of the occupant; and at least one seat belt retractor for applying tension to said seat belts in the winding-up direction.

Even when the occupant is subjected to strong impact produced by sudden stop or collision of an automobile when the wheelchair is put in the automobile, the occupant's body can be securely restrained to the wheelchair by the seat belts with tension in the winding-up direction of the seat belts, thereby ensuring the safety of the occupant. When the seat belts are put off for the normal use on the ground, the seat belt retractor automatically winds up and accommodates the seat belts, thereby releasing the occupant from the burden due to the seat belts so as to make the occupant to be comfortable and permitting the smooth movement of the occupant to get off the wheelchair.

In the wheelchair of the present invention, it is preferable that the number of the seat belt retractors is two or more.

The plurality of seat belt retractors further ensure the restraint of the occupant's body by the seat belts when receiving impact, thereby further ensuring the safety of the occupant.

In the wheelchair of the present invention, it is preferable that the seat belt retractor is mounted on the back side of the seat.

That is, the seat belt retractor is mounted on the back side of the seat of which strength is increased because of structure. Therefore, even when the occupant receives strong impact produced by sudden stop or collision of the automobile when the wheelchair is put in the automobile, the seat belt retractor securely applies tension to the seat belt in the winding-up direction without coming off the seat even when the seat is damaged, thereby restraining the occupant's body.

In the wheelchair of the present invention, it is preferable that the seat belt retractor has a locking mechanism which locks the movement of the seat belt in the withdrawing direction when tension exceeding a predetermined value is applied to the seat belt.

Accordingly, the withdrawal of the seat belt is locked when sudden tension is applied to the seat belt, thereby further ensuring the safety of the occupant.

In the wheelchair of the present invention, the seat has a monocoque structure in which a seating portion on which the occupant is seated, a backrest portion which receives the upper body of the occupant, and a headrest portion which is positioned on the upper end of the backrest portion and receives the head of the occupant are formed integrally.

Accordingly, the entire strength of the wheelchair is improved so as to increase the durability against impact and, in addition, the occupant's body can be reliably and securely restrained via the seat belt retractor and the seat belts.

In the wheelchair of the present invention, the monocoque structure includes a mounting portion which is located on the lower surface of said seating portion for mounting a main wheel shaft supporting portion supporting a main wheel shaft.

Accordingly, the entire strength of the wheelchair is further improved so as to increase the durability against impact, thereby further ensuring the safety of the occupant.

In the wheelchair of the present invention, the seat belt retractor is disposed near the main wheel shaft supporting portion and on the lower surface of the seating portion.

That is, the seat belt retractor can be attached to some of a lot of reinforcement members disposed near the main wheel shaft supporting portion having increased strength because of the reinforcement members. Therefore, the seat belt retractor securely locks the seat belts even when sudden tension is applied to the seat belts, thereby securely restraining the occupant's body.

In the wheelchair of the present invention, it is preferable that a rib is disposed on the backrest portion to extend along the longitudinal direction of the backrest portion.

The rib increases the strength of the backrest portion so that the upper body of the occupant of which movement is most drastic when receiving the impact can be reliably restrained.

It is preferable that the wheelchair of the present invention has a handle member which is disposed substantially horizontally in parallel with the backrest portion.

The handle member facilitates the pushing action and the pulling action of a care provider positioned behind the wheelchair and in addition, permitting the care provider to operate the wheelchair by only one hand when no occupant is seated, i.e., the wheelchair is vacant.

In the wheelchair of the present invention, it is preferable that an armrest portion for receiving an arm of the occupant and a mud guard covering the upper portion of the main wheel are formed integrally.

Accordingly, even when the wheelchair is structured to be compact by locating the main wheels near the seating portion, the occupant is prevented from touching tires of the main wheels, and the comfortable use is provided because the occupant can put his or her arms on the armrest portions comfortably.

In the wheelchair of the present invention, it is preferable that the seating portion and said backrest portion are arranged to have an angle of 90 degrees or more therebetween.

Accordingly, the occupant can be seated in a comfortable position such that the occupant leans his or her upper body on the backrest portion.

In the wheelchair of the present invention, it is preferable that the position of center-of-gravity of the wheelchair is higher than the main wheel shaft and lower than the center point of the lumbar of the occupant when the occupant is seated.

Accordingly, the center of gravity of the wheelchair when the occupant is seated is stable and the occupant can easily balance by moving his or her lumbar, thereby preventing the occupant from toppling down even when receiving impact.

### [Effects of the Invention]

According to the present invention, an occupant's body can be securely restrained even against a collision of an automobile when a wheelchair is put in the automobile, thereby ensuring the safety of the occupant.

### [Brief Explanation of the drawings]

Fig. 1 is an entire perspective view of a wheelchair according to the embodiment of the present invention, taken obliquely from above.
Fig. 2 is an entire perspective view of the wheelchair, taken obliquely from below.
Fig. 3 is an entire side view of the wheelchair.
Fig. 4 is an entire front view of the wheelchair.
Fig. 5 is an entire bottom view of the wheelchair.
Fig. 6 is an enlarged view of a buckle accommodation portion in a state that a common buckle is accommodated.

### [Best Modes for carrying out the Invention]

Hereinafter, an embodiment of the present invention will be described with reference to the attached drawings.

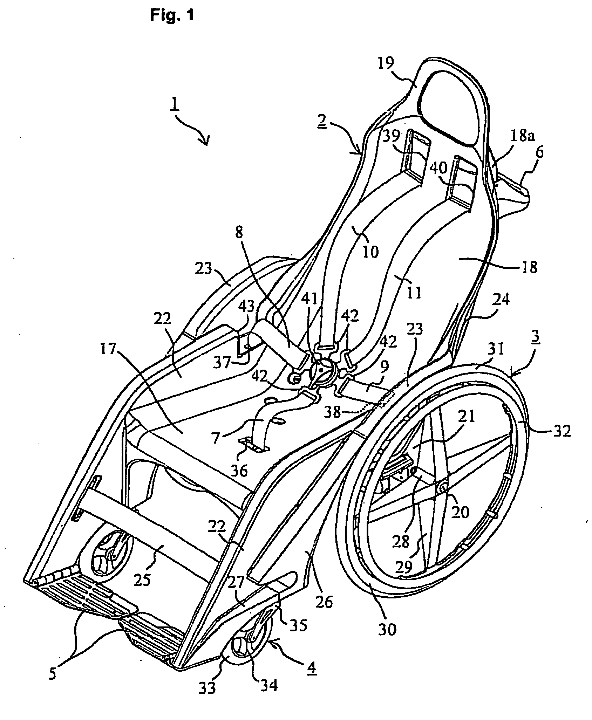

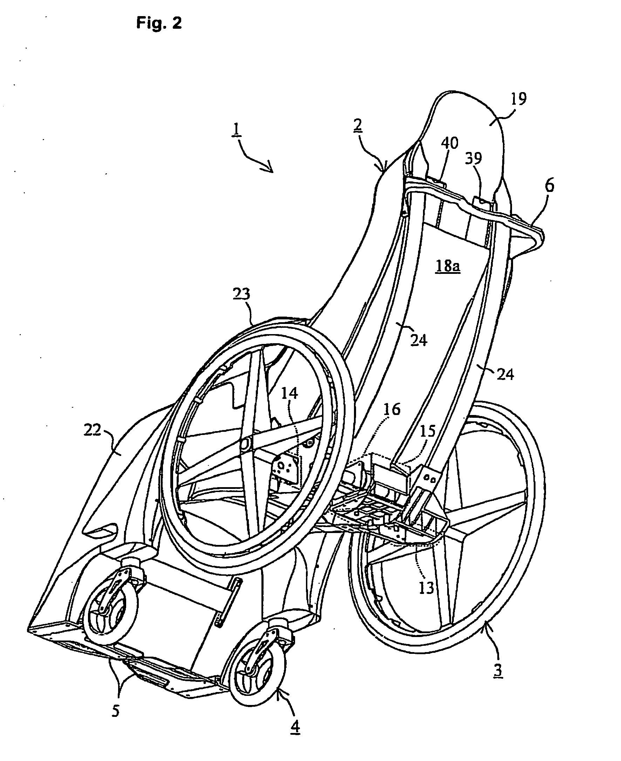

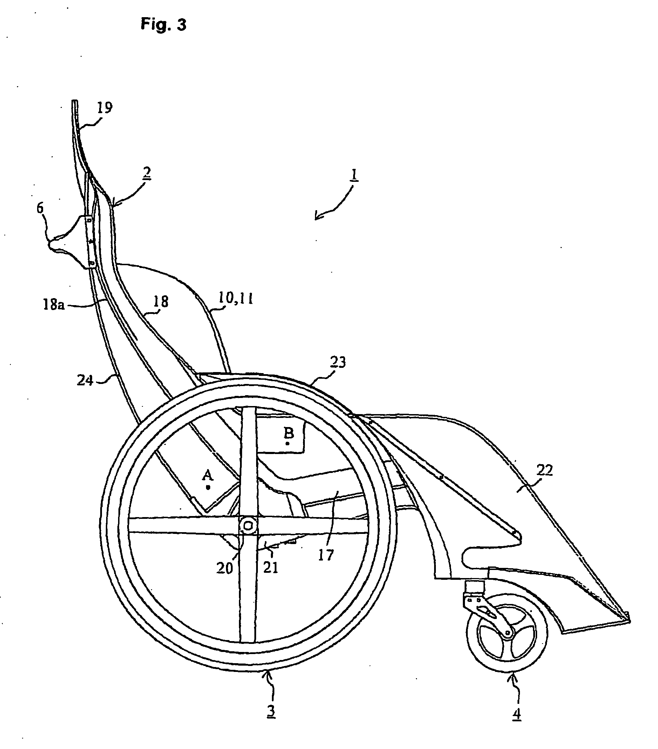

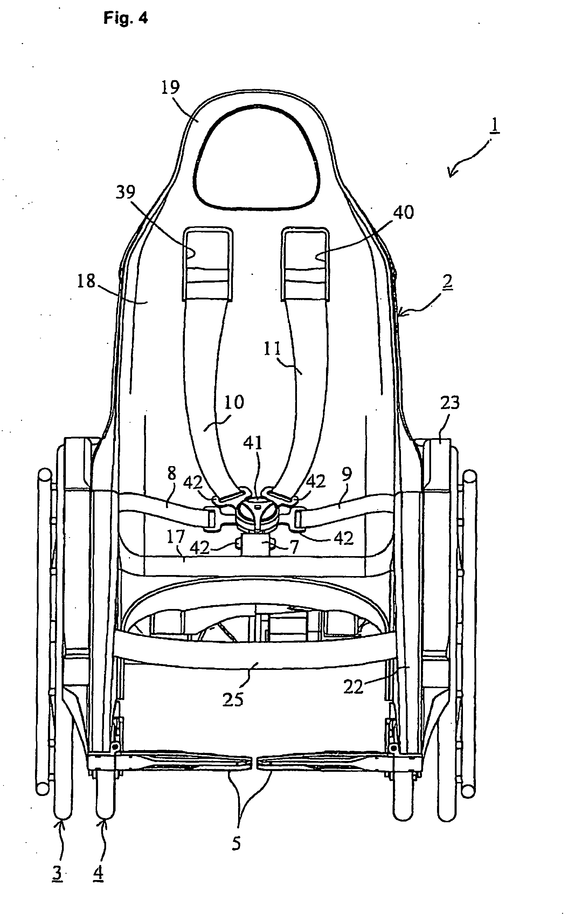

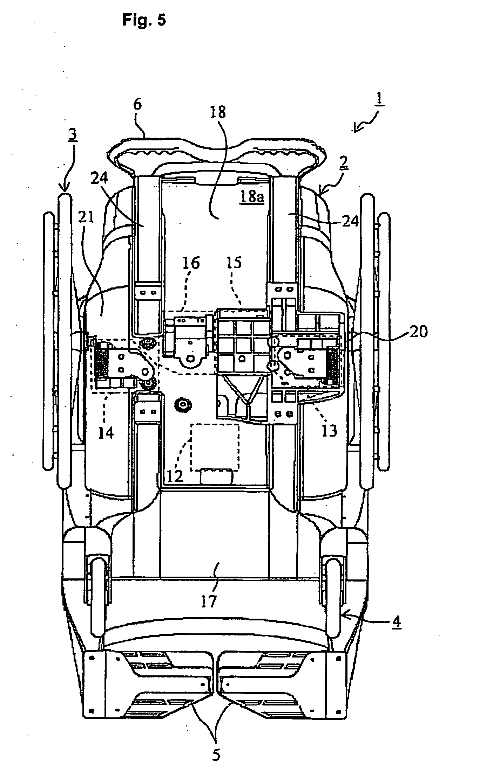

Fig. 1 is an entire perspective view of a wheelchair according to the embodiment of the present invention, taken obliquely from above, Fig. 2 is a perspective view of the same, taken obliquely from below, Fig. 3 is a side view of the same, Fig. 4 is a front view of the same, and Fig. 5 is a bottom view of the same. In Fig. 1, the wheelchair 1 according to this embodiment generally comprises a seat body 2, a pair of main wheels 3 provided on both lower sides at a rear portion of the seat body 2, a pair of front auxiliary wheels 4 provided on both lower sides at a front portion of the seat body 2, a pair of footrests 5 provided on both side at the front portion of the seat body 2, a handle 6 provided on the backside of the seat body 2, five seat belts 7, 8, 9, 10, and 11 which are withdrawn from corresponding portions of the seat body 2, and seat belt retractors 12, 13, 14, 15, and 16 (shown by broken lines in Fig. 2 and Fig. 5) connecting the respective seat belts 7 through 11 to the seat body 2.

First, the function of the wheelchair 1 will be schematically described. An occupant is seated in the seat body 2 and puts his or her feet on the footrests 5, whereby the occupant becomes in the normal seated state. Further from this state, the respective seat belts 7 through 11 are withdrawn form the respective seat belt retractors 12 through 16 and are suitably fastened, whereby the occupant's body can be securely restrained to the seat body 2. The occupant can move the wheelchair by himself or herself by rotating the main wheels 3 with his or her hands. A care provider can easily move the wheelchair 1 by grasping the handle 6 regardless of with or without the occupant in the wheelchair 1.

When the wheelchair 1 of this embodiment is put in and fixed to a cargo compartment of an automobile by an attachment as will be described later, the occupant's body can be securely restrained to the wheelchair 1. Accordingly, even when subjected to strong impact produced by sudden stop or collision of the automobile, the wheelchair can restrain the occupant's body sufficiently and thus ensures the safety of the occupant.

Hereinafter, the respective sections exhibiting the above functions will be described in detail.

First, the seat body 2 will be described. The seat body 2 comprises a seating portion 17 on which the occupant is seated, a backrest portion 18 which receives the upper body of the occupant, a headrest portion 19 which is positioned on the upper end of the backrest portion 18 and receives the head of the occupant, mounting portions (not shown) which are located on the lower surface of the seating portion 17 and to which main wheel shaft supporting portions 21 supporting the main wheels 3 and a main wheel shaft 20 are mounted by bolts or the like, side walls 22 which are positioned to extend obliquely from the both sides of the seating portion 17 to the front lower sides, and armrest portions 23 which are positioned on both sides at rear and upper portions of the side walls 22. These are all made of CFRP (Carbon-Fiber Reinforced Plastics, hereinafter referred to as "CFRP") and are formed integrally as a monocoque chair-like structure. It should be noted that the material is not limited to CFRP and may be other composite material such as GFRP (Glass-Fiber Reinforced Plastics), or resin material. All of the corners of the seat body 2 are curved to make the entire configuration to be composed of stream surfaces and streamlines. However, in a background example that is not an embodiment, the headrest portion 19 may not be formed in the monocoque structure and may be a separate structure which is telescopic or vertically shiftable relative to the other portion. Similarly, the armrest portions 23 may not be formed in the monocoque structure and may also be separate structures which are attached to the other portion by bolts or the like.

Since the seat body 2 receiving the occupant seated and functioning as a main structure member connecting the respective parts of the wheelchair 1 is formed in the monocoque structure, the entire strength of the wheelchair 1 is improved so as to increase the durability against impact and, in addition, the occupant's body can be reliably and securely restrained via the seat belt retractors 12 through 16 and the seat belts 7 through 11.

Since the seat body 2 is formed in a configuration composed of stream surfaces and streamlines, stress concentration is hardly generated even when being subjected to large load such as impact so that the structure has very high durability. Though the seat body 2 is preferably formed integrally by high-strength CFRP as mentioned above, the material for the seat body 2 is not limited thereto and, of course, the seat body 2 may be made of another high-strength material such as high-strength aluminum. As for the monocoque structure of the seat body 2, durability required to withstand impact is achieved when at least the seating portion 17, the backrest portion 18, and the headrest portion 19 are integrally formed. However, the durability is significantly improved in case of the monocoque structure in which the mounting portions to which the main wheel shaft supporting portions 21 are mounted are also formed integrally, like the wheelchair of this embodiment.

The structure of the backrest portion 18 has high strength to withstand large load from the seat belts 8, 9 at a collision when the wheelchair is put in the automobile. A rear surface 18a (opposite to the surface to be in contact with the back of the occupant) of the backrest portion 18 is provided with ribs 24 formed on the left and right sides thereof to extend in the vertical longitudinal direction, thereby further improving the strength of the backrest portion 18. Therefore, the upper body of the occupant of which movement is most drastic when receiving the impact can be reliably restrained. Particularly, the ribs 24 extending in the vertical direction as illustrated make the backrest portion 18 to be hardly bent even if large load is applied in the anteroposterior direction. That is, the backrest portion 18 is structured to be strong particularly against frontal impact. The illustrated ribs 24 have hollow box-like structure, but not limited thereto. The ribs 24 may be composed of angle bars or channel bars.

The seating portion 17 and the backrest portion 18 are arranged to have an angle of 90 degrees or more (in this embodiment, about 120 degree) therebetween so that the backrest portion 18 tilts backward as shown in Fig. 3. The angle of 90 degrees or more between the seating portion 17 and the backrest portion 18 enables the occupant to sit in a comfortable position such that the occupant can lean his or her upper body on the backrest portion.

By that ballasts (not shown) are placed on the respective portions in addition to that the backrest portion 18 is arranged to tilt backward, the weight balance is set such that the position of center-of-gravity of the wheelchair, when the occupant is seated, as indicated by a point A in Fig. 3 is higher than the main wheel shaft 20 and lower than the center point (indicated by a point B in Fig. 3) of the lumbar of the occupant. According to this structure, the center of gravity of the wheelchair 1 when the occupant is seated is stable and the occupant can easily balance by moving his or her lumbar, whereby the occupant hardly topples down even when receiving impact.

Returning to Fig. 1, the headrest portion 19 is positioned that the headrest portion 19 is in contact with the back of the head of the occupant when seated. A headrest mat, not shown, may be attached to the center of the headrest portion 19. In this case, the impact against the head is effectively absorbed even when receiving the impact due to a collision as mentioned above.

The main wheel shaft supporting portions 21 support the main wheel shaft 20 such that the main wheel shaft 20 projects laterally from the seat body 2, penetrates the main wheels 3, and rotatably supports the main wheels 3. The main wheel shaft supporting portions 21 bear most of the weight of the occupant and transmit the load to the main wheels 3 via the main wheel shaft 20. Therefore, reinforcement members are mostly concentrated on the main wheel shaft supporting portions 21 among the seat body 2.

The armrest portions 23 are portions on which the occupant puts on his or her arms and which are formed integrally with mud guards covering the upper portions of the main wheels 3, respectively. Therefore, even when the wheelchair 1 is structured to be compact by locating the main wheels 3 near the seating portion 17, the occupant is prevented from touching tires (as will be described later) of the main wheels 3, thereby providing safe and hygienic use and providing comfortable use because the occupant can put his or her arms on the armrest portions comfortably.

A leg belt 25 (for example, composed of an expandable belt, marketed as Magic-belt) is provided to extend between the side walls 22 in the lateral direction above the footrests 5. The leg belt 25 receives the calves of the occupant and supports to prevent the legs of the occupant from slipping down even when the occupant has weak legs.

Attachments 26 are fixed to the outer surfaces of rear portions of the side walls 22 by bolts, respectively. Each attachment 26 is provided with a slit 27 opening the front. A securing device fixed to the cargo compartment of the automobile (not shown) is attached to the slits 27, thereby securing the wheelchair 1 to the cargo compartment.

Now, the main wheels 3 will be described. Each main wheel 3 is provided with a bearing inside thereof (the bearing may be provided on the main wheel shaft 20 not with the main wheel 3) and comprises a hub 28 rotatably supported to the main wheel shaft 20, a plurality of (four, in this embodiment) spokes which extend radially from the hub 28, a rim 30 which connects the spokes and is disposed coaxially with the hub 28, a rubber tire 31 fitted to the outer periphery of the rim 30, and a push rim 32 which is an annular ring having substantially the same diameter as the rim 30 and is connected to and spaced apart from the rim 30 outwardly in the lateral direction. The occupant can operate the wheelchair 1 to move forward, move backward, turn left, and turn right by grasping the push rims 32 on both sides and rotating the push rims 32 to and fro.

Next, the front auxiliary wheels 4 will be described. Each front auxiliary wheel 4 comprises a wheel 34, a rubber tire 33 fitted to the outer periphery of the wheel 34, and a caster fork 35 to which the wheel 34 is rotatably supported. The caster fork 35 is supported at the lower end of the side wall 22 of the seat body 2 such that the caster fork 35 can rotate about a vertical shaft. The front auxiliary wheels 4 having the aforementioned structure support load on the front side of the wheelchair 1 and facilitate the smooth movement of the wheelchair 1 in the desired direction.

The footrests 5 will be described below. The footrests 5 are composed of flat plates which are supported pivotally in the vertical direction between the side walls 22 of the seat body 2. When the footrests 5 are lowered, the footrests 5 are stopped by stoppers, not shown, at a position that the footrests 5 face each other in the horizontal direction. The footrests 5 are installed in so-called flip-up style so as to allow free pivotal movement thereof above their horizontal positions. The legs of the occupant seated in the wheelchair are stable when the legs of the occupant are put on the footrests 5 which are lowered and fixed in the horizontal direction. When the occupant is about to get on and off the wheelchair 1, the occupant is allowed to stand directly on the ground near the seating portion 17 by lifting the footrests 5. This prevents the wheelchair 1 from falling if the occupant stands on the footrests 5.

The handle 6 will be described below. The handle 6 (handle member) is a rod-like member as shown in Fig. 2 and is arranged to extend substantially horizontally in parallel with the backrest portion 18. A generally-used conventional handle is composed of backrest pipes, i.e., a pair of cantilevered grippers disposed on the both rear sides of the backrest portion to extend in the anteroposterior horizontal direction. On the other hand, the handle 6 of this embodiment is formed in a C-like shape extending between the left side and the right side. Therefore, the care provider positioned behind the wheelchair 1 can grip more conveniently, thereby facilitating the pushing action and the pulling action. In addition, the handle can be gripped on the center line of the wheelchair 1 by one hand, thereby facilitating the handling of the wheelchair 1 when no occupant is seated, i.e., the wheelchair is vacant.

Hereinafter, the seat belts 7 through 11 and the seat belt retractors 12 through 16 will be described. The five seat belts 7 through 11 are allowed to be withdrawn through holes 36, 37, 38, 39, and 40 formed in the seat body 2 so that one of the seat belts can be withdrawn through a hole 36 formed at a central portion of the seating portion 17, two of the other seat belts can be withdrawn through holes 37, 38 formed near the armrest portions 23 of the respective side walls 22, the rest of the two seat belts can be withdrawn through two holes 39, 40 formed at the same level in the backrest portion 18, respectively. The seat belt 8 to be withdrawn through the hole 37 formed in the side wall 22 on the right side, as seen from the occupant, is provided at its end with a common buckle 41, while the other four seat belts 7, 9, 10, and 11 are provided at their ends with tongues 42 which can be latched to the common buckle 41.

When the seat belts 7 through 11 are withdrawn and the respective tongues 42 are latched to the common buckle 41, the seat belts 7 through 11 extend radially from the common buckle 41 as shown in Fig. 4. The occupant is restrained at his or her shoulders to the backrest portion 18 by the upper two seat belts 10 and 11, is restrained at his or her abdomen to the backrest portion 18 by the side two seat belts 8 and 9, and is restrained at his or her hips to the seating portion 17 by the lower one seat belt 7.

All of the seat belts 7 through 11 are connected to the seat body 2 via the seat belt retractors 12 through 16, respectively as shown in Fig. 2. The seat belt retractors 12 through 16 retract the respective seat belts 7 through 11 with light natural force and allow the respective seat belts 7 through 11 to be withdrawn for predetermined lengths. The seat belt retractors 12 through 16 always apply tension in the winding-up direction to the respective seat belts 7 through 11. Therefore, when the seated occupant puts on the seat belts 7 through 11, the seat belts 7 through 11 can restrain the occupant's body with suitable tension. When the seat belts 7 through 11 are unlatched from the common buckle 41 and thus become free, the seat belt retractors 12 through 16 wind up the respective seat belt 7 through 11 automatically. Moreover, the seat belt retractors 12 through 16 have locking mechanisms for locking the movement of the seat belts 7 through 11 in the withdrawing direction when sudden tension is applied to the respective seat belts 7 through 11.

Accordingly, when the wheelchair 1 is put in the automobile and strong impact is applied to the occupant's body due to sudden stop or collision of the automobile, the occupant's body can be securely restrained to the wheelchair 1 by the tension of the seat belts 7 through 11 in the winding-up direction, thereby ensuring the safety of the occupant. When further strong impact is applied, sudden tension is applied to the seat belts 7 through 11 so that the seat belts 7 through 11 are locked from being withdrawn from the seat belt retractors 12 through 16 by the locking mechanisms, whereby the occupant's body is securely restrained to the wheelchair 1 by the seat belts 7 through 11, thus ensuring the safety of the occupant. When the wheelchair 1 is brought down from the automobile and the seat belts 7 through 11 are put off, the seat belt retractors 12 through 16 automatically wind up and accommodate the seat belts 7 through 11, thereby releasing the occupant from the burden due to the seat belts 7 through 11 so as to make the occupant to be comfortable and permitting the smooth movement of the occupant to get off the wheelchair 1.

The number of the seat belt retractors must be two or more, not one. It is preferable that each of the seat belts is provided with a seat belt retractor just like this embodiment. In this case, the restraint of the occupant's body by the seat belt while receiving impact is further ensured, thereby further ensuring the safety of the occupant. Though a five-point-type restraint by five seat belts like this embodiment is preferable, a four- or less- point-type restraint may be used depend on the condition.

In the wheelchair 1 of this embodiment, the seat belt retractors 12 through 16 are concentrated and are placed near the main wheel shaft supporting portions 21 as shown in Fig. 5. Particularly, the seat belt retractors 13 through 16 are placed over the rear surface at the back (opposite side of the seating side) of the seat. The reinforcement members are concentrated on the rear surface and the main wheel shaft supporting portions 21 which have increased strength as mentioned above, and the seat belt retractors 12 through 16 located near the line of the main wheel shaft can be directly attached to the reinforcement members. Therefore, even when sudden tension is applied to the seat belts 7 through 11, the seat belt retractors 12 through 16 securely lock without coming off the seat body 2, thereby securely restraining the occupant's body.

The seat belt retractors 12 through 16 may be of any type among various types used for seats in automobiles having the aforementioned locking function and automatic winding function. In this embodiment, the seat belt retractors 12 through 16 have limiter mechanisms, not shown. The limiter mechanisms stop the automatic winding to stop the tongues 42 of the seat belts 7 through 11 at respective predetermined positions before the holes 36 through 40, thereby preventing the seat belts 7 through 11 from being retracted too much to make the seat belts 7 through 11 hard to be withdrawn or impossible to be withdrawn. Instead of the limiter mechanism, stoppers impossible to pass through the holes 36 through 40 may be attached to the tongues 42, respectively, thereby preventing the seat belts 7 through 11 from being retracted too much.

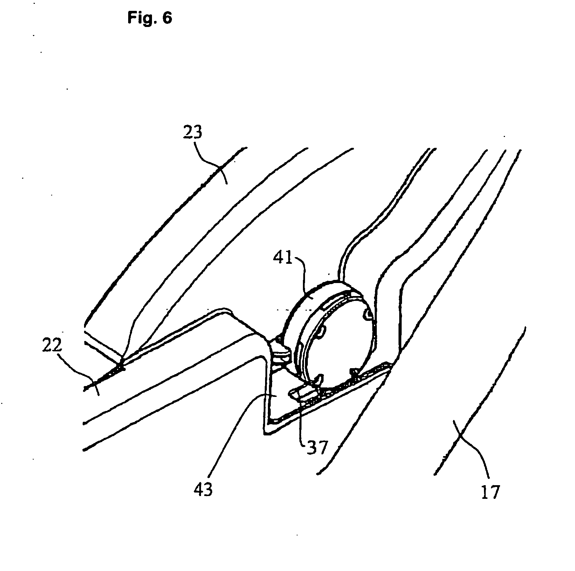

Near the hole 37 through which the right-side seat belt 8 with the common buckle 41, a buckle accommodation portion 43 is formed in the side wall 22 of the seat body 2 into a concave shape having such a size to allow the common buckle 41 to be fitted inside thereof. Accordingly, when the seat belts 7 through 11 are wound up and the common buckle 41 is retracted, the common buckle 41 is accommodated in the side wall 22 as shown in Fig. 6, thereby preventing the common buckle 41 from disturbing the occupant.

The wheelchair 1 of this embodiment having the aforementioned structure can provide more comfortable and safe use during normal use on the ground as compared to the conventional one. In addition, when the wheelchair 1 is put in the automobile, the occupant's body can be securely restrained as the case where the occupant's body is seated on a normal seat of the automobile and restrained by the automobile seat belt. That is, the wheel chair 1 of this embodiment has a function suitable for a case to be put in an automobile.

## Claims

1. A wheelchair comprising:
a seat (2) in which an occupant is to be seated;
seat belts (7-11) capable of restraining the body of the occupant; and
at least one seat belt retractor (12-16) for applying tension to said seat belts (7-11) in the winding-up direction,
wherein said seat (2) has a monocoque structure in which a seating portion (17) on which the occupant is seated, a backrest portion (18) which receives the upper body of the occupant, and a headrest portion (19) which is positioned on the upper end of the backrest portion (18) and receives the head of the occupant are formed integrally,
wherein the monocoque structure includes a mounting portion which is located on the lower surface of said seating portion (17) for mounting a main wheel shaft supporting portion (21) supporting a main wheel shaft (20), wherein said seat belt retractor (12-16) is disposed near said main wheel shaft supporting portion (21).

2. A wheelchair as claimed in claim 1, wherein the number of said seat belt retractors (12-16) is two or more.

3. A wheelchair as claimed in claim 1 or 2, wherein said seat belt retractor (12-16) is mounted on the back side of said seat (2).

4. A wheelchair as claimed in any one of claims 1 through 3, wherein said seat belt retractor (12-16) has a locking mechanism which locks the movement of said seat belt (7-11) in the withdrawing direction when tension exceeding a predetermined value is applied to said seat belt (7-11).

5. A wheelchair as claimed in any one of the preceding claims, wherein a rib (24) is disposed on the backrest portion (18) to extend along the longitudinal direction of the backrest portion (18).

6. A wheelchair as claimed in any one of the preceding claims, further comprising a handle member (6) which is disposed substantially horizontally in parallel with said backrest portion (18).

7. A wheelchair as claimed in any one of the preceding claims, wherein said seating portion (17) and said backrest portion (18) are arranged to have an angle of 90 degrees or more therebetween.

8. A wheelchair as claimed in any one of the preceding claims, wherein an armrest portion (23) for receiving an arm of said occupant and a mud guard covering the upper portion of a main wheel (3) are formed integrally.

9. A wheelchair as claimed in any one of the preceding claims, wherein the position (A) of center-of-gravity of the wheelchair (1) is higher than the main wheel shaft (20) and lower than the center point (B) of the lumbar of the occupant when the occupant is seated.

## Patentansprüche

1. Rollstuhl umfassend:
einen Sitz (2), in welchen sich ein Nutzer setzen kann;
Sitzgurte (7-11), welche in der Lage sind, den Körper des Nutzers zurückzuhalten; und
mindestens eine Sitzgurt-Aufrollvorrichtung (12-16), um einen Zug auf die Sitzgurte (7-11) in der Aufwickelrichtung auszuüben,
wobei der Sitz (2) eine Ganzschalenstruktur aufweist, bei welcher ein Sitzabschnitt (17), auf welchem der Nutzer sitzt, ein Rückenlehnenabschnitt (18), welcher den Oberkörper des Nutzers aufnimmt, und ein Kopflehnenabschnitt (19), welcher auf dem oberen Ende des Rückenlehnenabschnitts (18) angeordnet ist und den Kopf des Nutzers aufnimmt, integral ausgebildet sind,
wobei die Ganzschalenstruktur einen Montageabschnitt umfasst, welcher auf der unteren Oberfläche des Sitzabschnitts (17) zur Montage eines Hauptradwellenhalteabschnitts (21) angeordnet ist, der eine Hauptradwelle (20) hält, wobei die Sitzgurt-Aufwickelvorrichtung (12-16) in der Nähe des Hauptradwellenhalteabschnitts (21) angeordnet ist.

2. Rollstuhl nach Anspruch 1, **dadurch gekennzeichnet, dass** die Anzahl der Sitzgurt-Aufwickelvorrichtungen (12-16) zwei oder mehr beträgt.

3. Rollstuhl nach Anspruch 1 oder 2, **dadurch gekennzeichnet, dass** die Sitzgurt-Aufwickelvorrichtung (12-16) auf der Rückseite des Sitzes (2) angebracht ist.

4. Rollstuhl nach einem der Ansprüche 1 bis 3, **dadurch gekennzeichnet, dass** die Sitzgurt-Aufwickelvorrichtung (12-16) einen Verriegelungsmechanismus aufweist, welcher die Bewegung des Sitzgurts (7-11) in der Abwickelrichtung blockiert, wenn ein Zug, welcher einen vorbestimmten Wert überschreitet, auf den Sitzgurt (7-11) aufgebracht wird.

5. Rollstuhl nach einem der vorhergehenden Ansprüche, **dadurch gekennzeichnet, dass** eine Rippe (24) auf dem Rückenlehnenabschnitt (18) derart angeordnet ist, dass sie sich entlang der Längsrichtung des Rückenlehnenabschnitts (18) erstreckt.

6. Rollstuhl nach einem der vorhergehenden Ansprüche, **dadurch gekennzeichnet, dass** der Rollstuhl darüber hinaus ein Bedienteil (6) umfasst, welches im Wesentlichen horizontal parallel zu dem Rückenlehnenabschnitt (18) angeordnet ist.

7. Rollstuhl nach einem der vorhergehenden Ansprüche, **dadurch gekennzeichnet, dass** der Sitzabschnitt (17) und der Rückenlehnenabschnitt (18) derart angeordnet sind, dass sie einen Winkel von 90 Grad oder mehr dazwischen aufweisen.

8. Rollstuhl nach einem der vorhergehenden Ansprüche, **dadurch gekennzeichnet, dass** ein Armlehnenabschnitt (23) zur Aufnahme eines Arms des Nutzers und ein Schmutzschutz, welcher den oberen Abschnitt eines Hauptrads (3) überdeckt, integral ausgebildet sind.

9. Rollstuhl nach einem der vorhergehenden Ansprüche, **dadurch gekennzeichnet, dass** die Position (A) eines Schwerpunkts des Rollstuhls (1) höher als die Hauptradwelle (20) und tiefer als der Mittelpunkt (B) des Lendenbereichs des Nutzers liegt, wenn der Nutzer sitzt.

## Revendications

1. Fauteuil roulant comprenant :
un siège (2) dans lequel peut prendre place un occupant;
des ceintures de sécurité (7-11) capables de retenir le corps de l'occupant ; et
au moins un enrouleur de ceinture de sécurité (12-16) pour appliquer une tension audites ceintures de sécurité (7-11) dans le sens de l'enroulement,
dans lequel ledit siège (2) présente une structure monocoque dans laquelle sont formées de façon monobloc une partie formant siège (17) sur laquelle s'assoit l'occupant, une partie formant dossier (18) qui reçoit la partie supérieure du corps de l'occupant, et une partie formant appui-tête (19) qui est positionnée sur l'extrémité supérieure de la partie formant dossier (18) et qui reçoit la tête de l'occupant,
dans lequel la structure monocoque comprend une partie de montage située sur la surface inférieure de ladite partie formant siège (17) pour le montage d'une partie de support (21) d'axe de roue principale supportant un axe de roue principale (20),
dans lequel ledit enrouleur de ceinture de sécurité (12-16) est disposé à proximité de ladite partie de support (21) d'axe de roue principale.

2. Fauteuil roulant selon la revendication 1, dans lequel le nombre desdits enrouleurs de ceinture de sécurité (12-16) est égal ou supérieure à deux.

3. Fauteuil roulant selon la revendication 1 ou 2, dans lequel ledit enrouleur de ceinture de sécurité (12-16) est monté sur le côté arrière dudit siège (2).

4. Fauteuil roulant selon l'une quelconque des revendications 1 à 3, dans lequel ledit enrouleur de ceinture de sécurité (12-16) présente un mécanisme de verrouillage qui bloque le déplacement de ladite ceinture de sécurité (7-11) dans le sens de retrait lorsqu'une tension qui dépasse une valeur prédéterminée est appliquée à ladite ceinture de sécurité (7-11).

5. Fauteuil roulant selon l'une quelconque des revendications précédentes, dans lequel une nervure (24) est disposée sur la partie formant dossier (18) pour s'étendre le long de la direction longitudinale de la partie formant dossier (18).

6. Fauteuil roulant selon l'une quelconque des revendications précédentes, comprenant en outre un organe formant poignée (6) disposé sensiblement horizontalement, parallèle à ladite partie formant dossier (18).

7. Fauteuil roulant selon l'une quelconque des revendications précédentes, dans lequel ladite partie formant siège (17) ainsi que ladite partie formant dossier (18) sont agencées pour avoir un angle égale ou supérieure à 90° entre elles.

8. Fauteuil roulant selon l'une quelconque des revendications précédentes, dans lequel sont formées de façon monobloc une partie formant accoudoir (23) pour recevoir un bras dudit occupant ainsi qu'un garde-boue recouvrant la partie supérieure d'une roue principale (3).

9. Fauteuil roulant selon l'une quelconque des revendications précédentes, dans lequel la position (A) du barycentre du fauteuil roulant (1) est plus haut que l'axe de roue principale (20) et à un niveau plus bas que le point central (B) du lombaire de l'occupant lorsque l'occupant est assis.
